# EUROPEAN PATENT APPLICATION

(11) **EP 3 305 758 A1**
(43) Date of publication of application: **11.04.2018**
(21) Application number: 16803022.9
(22) Date of filing: 13.05.2016
(51) Int. Cl.: C07C 227/18, A61K 51/00, B01J 20/22, C07B 59/00, C07C 229/48

(54) **DEVICE AND METHOD FOR PRODUCING RADIOACTIVELY LABELED COMPOUND**

(30) Priority: 05.06.2015 JP 2015115179
(71) Applicant: Nihon Medi-Physics Co., Ltd., Tokyo 136-0075 (JP)
(72) Inventor: NAKAMURA, Daisaku, Tokyo 136-0075 (JP); TOYAMA, Masahito, Tokyo 136-0075 (JP); AKAMA, Kei, Tokyo 136-0075 (JP); EHARA, Hideaki, Tokyo 136-0075 (JP)
(74) Representative: TBK
(86) International application number: PCT/JP2016/064396
(87) International publication number: WO 2016/194586

(57) **Abstract**

An apparatus for producing a radiolabeled compound (100) is a production apparatus which produces a radiolabeled compound by introducing a radioisotope into a non-radioactive labeling precursor compound. The production apparatus (100) includes a solid phase extraction unit (10) in which a specific process which is a reaction of an intermediate compound, a purification of the intermediate compound, or a purification of the radiolabeled compound is carried out; and a cooling unit (20) that cools the solid phase extraction unit (10), when the specific process is carried out.

## Description

### TECHNICAL FIELD

This invention relates to an apparatus for producing a radiolabeled compound, and a method for producing the same.

### BACKGROUND ART

Radiolabeled compound is a compound labeled with a radioisotope, and is produced typically through a process of introducing a radioisotope (nuclide) into a predetermined labeling precursor compound. The radiolabeled compound is typically used for radioactive medicines.

Patent Literature 1 describes a production apparatus used for producing an organic compound such as radiolabeled compound.

Patent Literature 1 also describes a method for producing [¹⁸F]1-amino-3-fluorocyclobutanecarboxylic acid (referred to as [¹⁸F]FACBC, hereinafter), a kind of radiolabeled compound, using the above-described production apparatus.

For the production of [¹⁸F]FACBC, for example, as described in Patent Literature 1, a production apparatus having a solid phase extraction column in which a deprotection reaction is carried out and a purification column that purifies [¹⁸F] FACBC is used.

As a known method of producing [¹⁸F]FACBC, for example, as described in Patent Literature 2, a method having a radio fluorination step that introduces radioactive fluorine into a labeling precursor compound; a deprotection step (de-esterification step) that deprotects (de-esterify) the intermediate compound produced in the radio fluorination step using an alkaline solution; and an amino deprotection step that deprotects the amino protective group for the compound obtained in the deprotection step.

When the above-described production apparatus is used, the deprotection step (de-esterification step) is carried out in the solid phase extraction column, and the purification of [¹⁸F] FACBC in the purification column takes place subsequent to the amino deprotection step.

### RELATED DOCUMENT

### PATENT DOCUMENT

[Patent Document 1] JP-A-2014-201571
[Patent Document 2] WO2007/132689, pamphlet

### SUMMARY OF THE INVENTION

### PROBLEM TO BE SOLVED BY THE INVENTION

The present inventors newly found that, in the production of [¹⁸F]FACBC, when the temperature of the solid phase extraction column becomes high during the deprotection step carried out by bringing it into contact with the alkaline solution, a part of packing material in the column could leaked into the eluate, to thereby clog the purification column, and made it unable to feed the reaction liquid. This trouble can occur not only during the production of [¹⁸F]FACBC, but also during production or purification of the radiolabeled compound as well, caused by the temperature of the solid phase extraction unit such as the solid phase extraction column becomes high. Even for the case where the purification column is not used, it is anticipated that the packing material may be eluted and get into the final product, to thereby degrade the purity of the final product, or to complicate the purification.

This invention was conceived in consideration of the above-described problem, and provides an apparatus for producing a radiolabeled compound, and a method for producing a radiolabeled compound, capable of suppressing troubles that would occur as a result of that the temperature of the solid phase extraction unit becomes high during the reaction of the intermediate compound, a purification of the intermediate compound, or the purification of the radiolabeled compound, by solid phase extraction method.

### MEANS FOR SOLVING THE PROBLEM

According to this invention, there is provided an apparatus for producing a radiolabeled compound which produces a radiolabeled compound by introducing a radioisotope into a non-radioactive labeling precursor compound, the apparatus includes:
a solid phase extraction unit in which a specific process which is a reaction of an intermediate compound, a purification of the intermediate compound, or a purification of the radiolabeled compound is carried out; and a cooling unit that cools the solid phase extraction unit, when the specific process is carried out.

According to this invention, there is also provided a method for producing a radiolabeled compound for producing a radiolabeled compound by introducing a radioisotope into a non-radioactive labeling precursor compound, the method includes performing a specific process in a solid phase extraction unit holding an intermediate compound or the radiolabeled compound retained therein, while locally cooling the solid phase extraction unit, the specific process being any one of a reaction of the intermediate compound, a purification of the intermediate compound, or a purification of the radiolabeled compound.

### EFFECT OF THE INVENTION

According to this invention, it is made possible to suppress troubles that would occur as a result of that the temperature of the solid phase extraction unit becomes high during the reaction of the intermediate compound, the purification of the intermediate compound, or the purification of the radiolabeled compound, by solid phase extraction method.

### BRIEF DESCRIPTION OF DRAWINGS

[FIG. 1] A schematic diagram of an apparatus for producing a radiolabeled compound according to a first embodiment.
[FIG. 2] A front elevation of an apparatus for producing a radiolabeled compound according to a second embodiment.
[FIG. 3] A perspective view illustrating an exemplary channel cartridge of the apparatus for producing a radiolabeled compound according to the second embodiment.
[FIG. 4] FIG. 4 (a) and FIG. 4 (b) are drawings illustrating a cooling unit of the apparatus for producing a radiolabeled compound according to the second embodiment, wherein FIG. 4 (a) is a front elevation, and FIG. 4(b) is a plan view.
[FIG. 5] FIG. 5 (a) and FIG. 5 (b) are drawings illustrating a cooling unit of the apparatus for producing a radiolabeled compound according to the second embodiment, wherein FIG. 5 (a) is a right side elevation, and FIG. 5(b) is a left side elevation of a compressed air feed pipe supporting bracket.
[FIG. 6] FIG. 6 (a) and FIG. 6 (b) are drawings illustrating a cover, and a periphery thereof, of the apparatus for producing a radiolabeled compound according to the second embodiment, wherein FIG. 6(a) is a front elevation, and FIG. 6(b) is a drawing illustrating a side geometry and a side cross-sectional geometry.
[FIG. 7] A drawing illustrating a block configuration of the apparatus for producing a radiolabeled compound according to the second embodiment.
[FIG. 8] A front elevation illustrating a solid phase extraction unit and a heat sink of the apparatus for producing a radiolabeled compound according to a modified example of the second embodiment.
[FIG. 9] A drawing for explaining an apparatus for producing a radiolabeled compound according to a third embodiment.
[FIG. 10] A time chart illustrating exemplary temperature changes of the solid phase extraction units of the apparatuses for producing a radiolabeled compound according to the third embodiment and a comparative embodiment.
[FIG. 11] A drawing illustrating a relation between temperature of the solid phase extraction unit, and the amount of silicon eluted from the solid phase extraction unit, in the process of producing the radiolabeled compound.
[FIG. 12] A front elevation of a cooling unit of the apparatus for producing a radiolabeled compound according to a modified example.

### DESCRIPTION OF THE EMBODIMENTS

The above and other objects, advantages and features of this invention will be more apparent from the following description of certain preferred embodiments taken in conjunction with the accompanying drawings.

Embodiments of this invention will be explained referring to the attached drawings. In all drawings, all similar constituents will be given same reference numerals or symbols, so as to suitably avoid repetitive explanations.

### [First Embodiment]

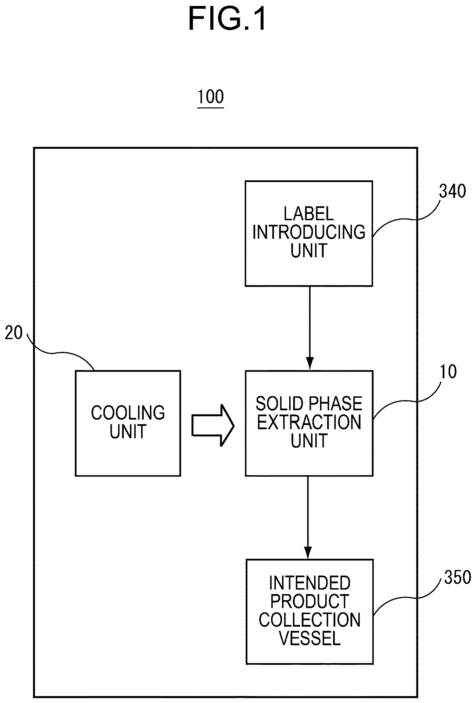

FIG. 1 is a schematic drawing illustrating an apparatus for producing a radiolabeled compound 100 (simply referred to as "production apparatus 100", hereinafter) according to the first embodiment.

As illustrated in FIG. 1, the production apparatus 100 is an apparatus for producing a radiolabeled compound which produces a radiolabeled compound by introducing a radioisotope into a non-radioactive labeling precursor compound, and has a solid phase extraction unit 10 in which a specific process is carried out, and a cooling unit 20 that cools the solid phase extraction unit 10, when the specific process is carried out. The specific process is a reaction of an intermediate compound, a purification of the intermediate compound, or a purification of the radiolabeled compound.

Among them, the reaction of the intermediate compound includes hydrolysis reaction such as de-esterification. The hydrolysis reaction includes deprotection reaction.

In this invention, the "intermediate compound" means an intermediate product obtained when the reaction, for producing an intended radiolabeled compound from the labeling precursor compound using the production apparatus 100, is performed by multiple-step, and in which a radioisotope is introduced. For example, where the intended radiolabeled compound has a substituent such as hydroxy group, carboxy group or amino group, which are active against the reaction that introduces the radioisotope, the labeling precursor compound will be understood to be a compound having an elimination group to be substituted by the radioisotope, and a protective group protecting these active groups; and the intermediate compound will be understood to be a compound having a radioisotope-containing substituent and a protective group.

Systems of cooling of the cooling unit 20 are not specifically limited, and may typically be a system using circulating water for cooling (water-cooled system), may be a system using a Peltier element for cooling, and may be a system using cold air as described later in other embodiments.

The radiolabeled compound produced by using the production apparatus 100 is preferably, but not specifically limited to, an organic compound. The radioisotope is exemplified by, but not specifically limited to, ¹⁸F or ¹¹C.

The solid phase extraction unit 10 is a column or a cartridge packet with a stationary phase having a granular or other shapes, suitable as a chromatographic packing. The packing material used for the column or cartridge is exemplified by silica gel-based packing material, resin-based packing material, ion exchange packing material, florisil-based packing material, and alumina-based packing material, wherein a solid phase carrier to which a silyl group is bonded is preferable.

The intermediate compound or radiolabeled compound subjected to the reaction or purification in the solid phase extraction unit 10 is preferably an organic compound, although the types of which not specifically limited.

Although types of reagents used for the reaction or purification in the solid phase extraction unit 10 are not specifically limited so long as they are liquid, alkali is preferably used for the case where the solid phase extraction unit 10 employs the solid phase carrier to which a silyl group is bonded. The alkali is exemplified by hydrates or alkoxides of alkali metals. These alkalis are used after dissolved into water or alcohol. Specific examples of the alkali include aqueous sodium hydroxide solution and sodium methoxide solution in methanol.

As described above, the solid phase extraction unit 10 typically has the solid phase carrier to which a silyl group is bonded, and the specific process is carried out at the solid phase extraction unit 10 in the presence of alkali.

The production apparatus 100 further has a label introducing unit 340 that introduces a radioisotope into the labeling precursor compound, and an intended product collection vessel 350 that collects the intended product to be produced.

The method for producing a radiolabeled compound according to this embodiment is a method for producing a radiolabeled compound by introducing a radioisotope into a non-radioactive labeling precursor compound includes performing a specific process in a solid phase extraction unit 10 holding an intermediate compound or the radiolabeled compound retained therein, while locally cooling the solid phase extraction unit 10, the specific process being any one of a reaction of the intermediate compound, a purification of the intermediate compound, or a purification of the radiolabeled compound.

Here, "locally cooling the solid phase extraction unit 10" means that a partial region, including the solid phase extraction unit 10, in the production apparatus 100 (but not the entire region of the production apparatus 100) is selectively cooled.

In the case that the production apparatus 100 has a cabinet (enclosure), for example, as described later in the second embodiment, the phrase can also mean that a partial region of the cabinet, including the solid phase extraction unit 10 (but not the entire region of the cabinet), is selectively cooled.

In the case that the production apparatus 100 has a heating unit for any process which performed under a heating condition, the phrase can even mean that the solid phase extraction unit 10 is cooled by the cooling unit 20, so that the heating unit will not be substantially cooled by the cooling effect of the cooling unit 20.

According to this embodiment, it can be suppressed that the temperature of the solid phase extraction unit 10 becomes high, as a result of cooling of the solid phase extraction unit 10 by the cooling unit 20. Accordingly, it is made possible to suppress troubles that would occur by a part of the packing material of the solid phase extraction unit 10 getting into the eluent due to the temperature of the solid phase extraction unit 10 becoming high during the process of the reaction of the intermediate compound, the purification of the intermediate compound, or the purification of radiolabeled compound, by solid phase extraction method. For example, in the case that the production apparatus 100 has a purification column, the purification column may be suppressed from being clogged.

### [Second Embodiment]

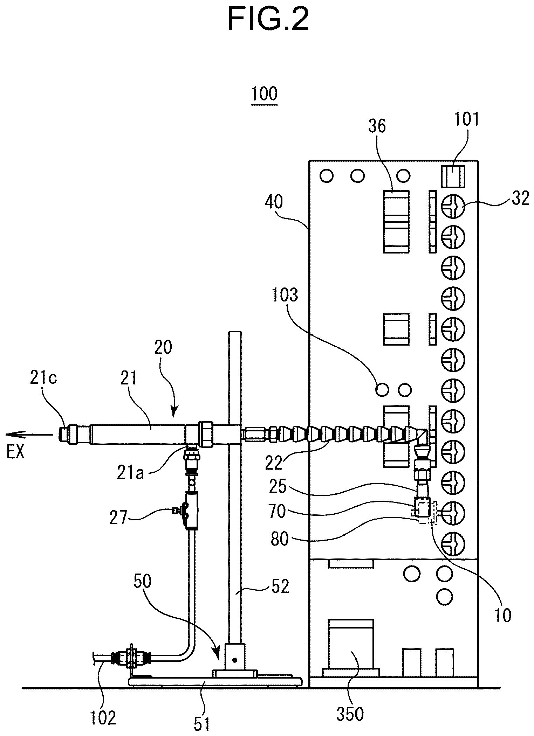

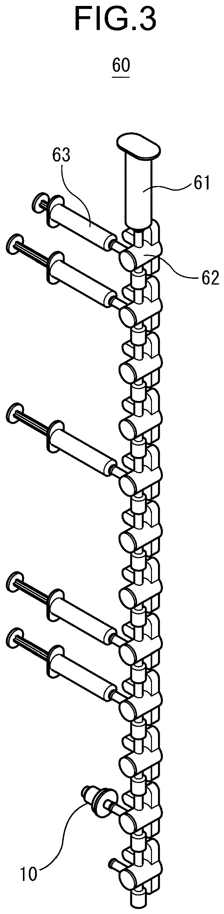

FIG. 2 and FIG. 3 are drawings for explaining the production apparatus 100 of the second embodiment. FIG. 2 is a front elevation of the production apparatus 100, and FIG. 3 is a perspective view illustrating an exemplary channel cartridge 60 owned by the production apparatus 100.

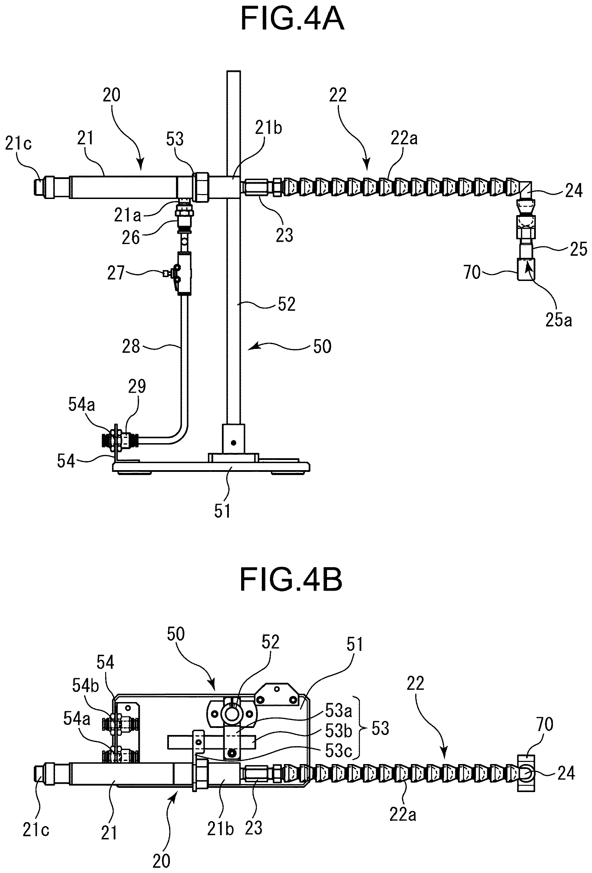

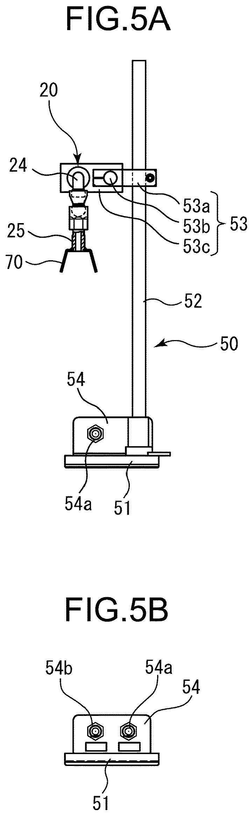

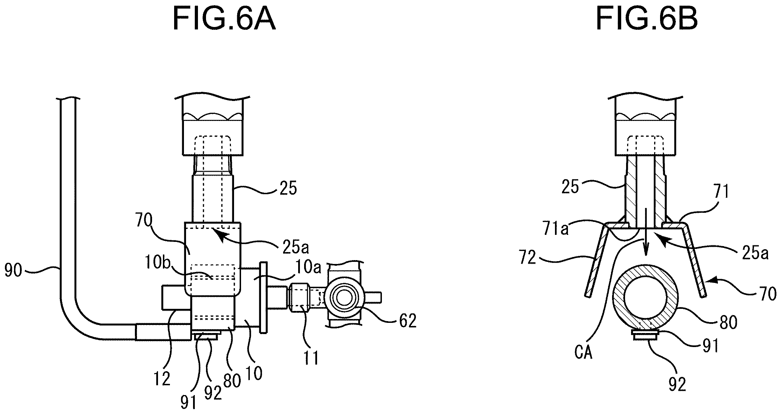

FIG. 4(a) to FIG. 6(b) are drawings for explaining the cooling unit 20 owned by the production apparatus 100. FIG. 4(a), FIG. 4(b) and FIG. 5(a) illustrate the cooling unit 20 and a support stand 50 that supports the cooling unit 20, wherein FIG. 4 (a) is a front elevation, FIG. 4(b) is a plan view, and FIG. 5 (a) is a right side elevation. FIG. 5 (b) is a left side elevation illustrating a compressed air feed pipe support bracket 54, owned by the support stand 50. FIG. 6 (a) and FIG. 6(b) are drawings illustrating a cover 70 owned by the production apparatus 100 and the periphery thereof, wherein FIG. 6(a) is a front elevation, and FIG. 6(b) is a drawing illustrating a side geometry and a side cross-sectional geometry.

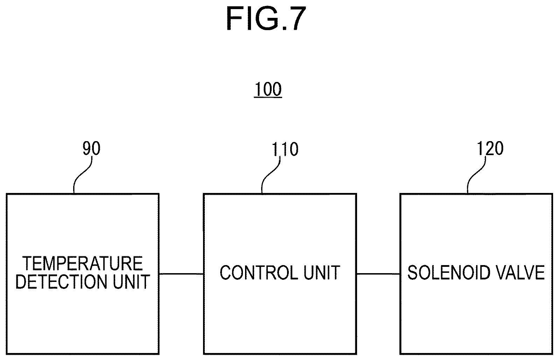

FIG. 7 is a drawing illustrating a block configuration of the production apparatus 100 according to the second embodiment.

The production apparatus 100 of this embodiment is arranged to be applicable to synthesis and purification of a variety of compounds.

The production apparatus 100 has a control unit 110 (FIG. 7) that controls operations of the production apparatus 100. The production apparatus 100 is designed so as to automatically carry out processes necessary for producing a predetermined radiolabeled compound, and to automatically produce predetermined radiolabeled compound, by controlling the operation of individual constituents of the production apparatus 100 under the control of the control unit 110. More specifically, by controlling the operation of the electric components including the motor and the like by the control unit 110, such predetermined radiolabeled compound may be produced automatically.

As illustrated in FIG. 2, the production apparatus 100 has a cabinet 40 to which a variety of constituents are mounted. In the cabinet 40, constituent elements corresponding to the type of compound to be produced using the production apparatus 100, the method of production, and the like are mounted.

The way of arrangement of the individual constituents in the production apparatus 100 is not specifically limited. For example, the individual constituents may be arranged vertically or may be arranged side by side in the horizontal direction or may have a hybrid arrangement of vertically arranged constituents and horizontally arranged constituents.

In general, the production apparatus 100 has a plurality of three-way cocks on a flow channel for a liquid used in the process of producing the radiolabeled compound. The three-way cocks may be detachably provided to the cabinet 40, or may be provided fixedly in a non-detachable manner.

As an example, in the production apparatus 100, the channel cartridge 60 shown in FIG. 3 is provided detachably.

In this case, there is provided a cartridge holder 101 that supports a liquid reservoir 61 of the channel cartridge 60 (FIG. 3), at the front face of an upper portion of the cabinet 40.

The cabinet 40 has a plurality of valve holders 32 provided to the front face thereof. Each valve holder 32 is designed so as to accept and hold a handle of the three-way cock 62 owned by the channel cartridge 60. Inside the cabinet 40, there are provided motors corresponding to the individual valve holders 32, with the rotating shafts of motors respectively connected to the valve holders 32. As each motor drives, the valve holder 32 rotates, also the three-way cock 62 held by the valve holder 32 rotates, to thereby switch the flow channel formed by the channel cartridge 60.

Operationally controlled components (electric components) such as motors provided in the cabinet 40 are controlled by control signals output from the control section 110. Accordingly, operations such as switching of the flow channel can be performed automatically under the control of the control unit 110.

For example, a syringe mounting portion 36 is provided on the front face of the cabinet 40 so that a syringe (not shown) can be attached to the front face of the cabinet 40. In this case, the cabinet 40 is provided with a syringe drive mechanism for moving the plunger of the syringe with a motor, a spring, or the like, and it is possible to automatically discharge the liquid from the syringe and suck the liquid into the syringe.

There may be an input-output port 103 provided to the front face of the cabinet 40. The input-output port 103 is a port through which the liquid used in the production process of the radiolabeled compound is input or output. Elements that perform various processes to the liquid may be mounted at the behind of the input-output port. For example, an element that controls temperature of the liquid, or a pump that elevates pressure of the liquid may be mounted.

The channel cartridge 60 illustrated in FIG. 3 forms at least a part of the flow channel of the liquid used in the process of producing the radiolabeled compound.

The channel cartridge 60 has, for example, a plurality of three-way cocks 62 connected to each other, syringes 63 attached to the three-way cocks 62, a column of, for example, the solid phase extraction unit 10 attached to one of the three-way cock 62, and a liquid reservoir 61 connected to the upper side of the topmost three-way cock 62.

For instance, the channel cartridge 60 is designed to be detachable on the front face of the cabinet 40. In this case, the channel cartridge 60 is held on the front face of the cabinet 40, with handles of individual three-way cocks 62 supported by the individual valve holders 32 provided to the front face of the cabinet 40, and with the liquid reservoir 61 supported by the cartridge holder 101 provided to the upper portion of the front face of the cabinet 40.

The channel cartridge 60 may, however, be fixed to the cabinet 40 in an undetachable manner.

Note that all configurations such that the channel cartridge 60 has the liquid reservoir 61 at the top, the position and number of illustrated solid phase extraction unit 10 and the syringe 63, the number of the three-way cocks 62, and distance between the adjacent three-way cocks 62, etc. are all illustrative, and may be modified according to specific demands.

The production apparatus 100 contains flow channel forming members such as unillustrated plastic tubes, which are suitably connected to the three-way cocks 62, the solid phase extraction unit 10, and the input-output port 103 to form the flow channel.

Preferred examples of the radiolabeled compound produced using the production apparatus 100 include the radiolabeled compound with a short life span used for nuclear medicine inspection using PET (positron emission tomography) or SPECT (single photon emission computed tomography). The production apparatus 100 may, however, be used for producing other types of radiolabeled compounds.

As illustrated in FIG. 2, also in this embodiment, the production apparatus 100 has a solid phase extraction unit 10 in which the specific process is carried out, and the cooling unit 20 that cools the solid phase extraction unit 10, when the specific process is carried out.

In this embodiment, the cooling unit 20 contains a cold air blower that cools the solid phase extraction unit 10 with cold air.

As illustrated in FIG. 4 (a), the cold air blower is, for example, designed to have a vortex tube 21.

The vortex tube 21 is a component capable of outputting compressed air introduced therein, while dividing it into cold air and hot air, and has an introduction unit 21a through which the compressed air is introduced, a cold air output unit 21b through which the cold air is blown out, and a hot air output unit 21c through which the hot air is blown out.

The vortex tube 21 is formed into a tubular shape elongated in one direction, has a cold air output unit 21b formed at one end thereof (the right end in FIG. 4 (a)), has a hot air output unit 21c formed at the other end (the left end in FIG. 4(a)), and has an introduction unit 21a formed on the outer circumference at the intermediate portion between both ends of the vortex tube 21.

When the compressed air is introduced into the vortex tube 21 through the introduction unit 21a, the cold air is blown out through the cold air output unit 21b toward one side (rightward in FIG. 4 (a)) in the longitudinal direction of the vortex tube 21, and hot air is blown out through the hot air output unit 21c towards the other side (leftward in FIG. 4(a)) in the longitudinal direction of the vortex tube 21.

Here, as illustrated in FIG. 2, the vortex tube 21 is disposed so that the hot air output unit 21c blows out the hot air towards the direction opposite to the solid phase extraction unit 10 with reference to the introduction unit 21a. With this design, the solid phase extraction unit 10 is prevented from being heated by the hot air blown out through the hot air output unit 21c.

To the introduction unit 21a of the vortex tube 21, compressed air is fed from an unillustrated supply source of the compressed air, through the compressed air feed pipe 102 (FIG. 2), a joint 29, a compressed air feed pipe 28, a joint 26 and so forth. The compressed air feed pipe 28 has provided thereto a speed controller 27 that controls the flow rate of the compressed air to be fed to the vortex tube 21, to thereby control cooling power of the vortex tube 21. When the flow rate of compressed air to be fed to the vortex tube 21 increases as a result of control operation by the user made on the speed controller 27, the cooling power of the vortex tube 21 increases, meanwhile when the flow rate of compressed air to be fed to the vortex tube 21 decreases, the cooling power of the vortex tube 21 decreases.

The cold air blower includes, for example, a flexible tube 22 that is connected to the cold air output unit 21b of the vortex tube 21 via a joint 23, and the flexible tube 22 includes an outlet port 25a for discharging cold air blown out from the cold air output unit 21b to the outside is formed on the tip end side of the flexible tube 22.

The flexible tube 22 has a plurality of hollow link components 22a, and is formed by coupling these link components 22a in series. Every adjacent link components 22a are coupled by a spherical joint in a bendable to each other. Thus, the flexible tube 22 is bendable as a whole with ease.

More specifically, for example, as illustrated in FIG. 4(a), an outlet pipe 25 is connected to the tip end of the flexible tube 22 via an L-shaped joint 24, and an outlet port 25a is formed at the tip end of the outlet pipe 25. Therefore, the cold air blown out from the cold air output unit 21b is allowed to pass inside the joint 23, inside the flexible tube 22, inside the L-shaped joint 24, and inside outlet pipe 25 in this order, and is discharged to the outside through the outlet port 25a. Note, as illustrated in FIG. 4(a), that a short flexible tube 22 may also be connected between the L-shaped joint 24 and the outlet pipe 25.

Being provided with the flexible tube 22 as described above, the cold air blower becomes possible to easily adjust the position at which the cold air is blown outward, and the direction of cold air blown out.

The production apparatus 100 has a support stand 50 that supports the cooling unit 20. For example, with the support stand 50 disposed alongside the cabinet 40, the solid phase extraction unit 10 may be cooled by the cooling unit 20 supported by the support stand 50.

The support stand 50 is, for example, has a flat base 51 that is disposed on a floor or the like, a support post 52 provided so as to rise up from the base 51, a support part 53 that is fixed to the support post 52 and holds the cooling unit 20, and a compressed air feed pipe support bracket 54 that holds a joint portion of the compressed air feed pipe 102 and the compressed air feed pipe 28 by holding the joint 29.

As illustrated in FIG. 4(b), the support part 53 is, for example, designed to have a first bracket 53a, a rod-like second bracket 53b, and a third bracket 53c.

The first bracket 53a is fixed to the support post 52, and holds the second bracket 53b in such a way possible to adjust the position of the second bracket 53b relative to the first bracket 53a in the longitudinal direction of the second bracket 53b.

Meanwhile, the third bracket 53c holds the vortex tube 21, and also holds the second bracket 53b. The third bracket 53c may hold the second bracket 53b in such a way possible to adjust the position of the second bracket 53b relative to the third bracket 53c in the longitudinal direction of the second bracket 53b.

In this state, the rod-like second bracket 53b is disposed in parallel with the vortex tube 21. Hence, by adjusting the position of the second bracket 53b relative to the first bracket 53a, or, the position of the second bracket 53b relative to the third bracket 53c, it becomes possible to adjust the position of the vortex tube 21 relative to the support post 52, in the longitudinal direction of the vortex tube 21.

The fixing position of the support part 53 relative to the support post 52 is freely adjustable. By adjusting the fixing position of the support part 53 relative to the support post 52, it becomes possible to freely adjust the height position of the vortex tube 21 supported by the support stand 50.

Only one cooling unit 20, or a plurality of cooling units 20 may be supported by the support stand 50.

For an exemplary case where a plurality of cooling units 20 are supported by the support stand 50, the plurality of cooling units 20 are respectively supported by the support post 52 through the support parts 53 at different levels of height.

The compressed air feed pipe supporting bracket 54 is, for example, made so as to hold the joint portions between the compressed air feed pipes 102 and the compressed air feed pipes 28 that are corresponded to two cooling units 20. That is, as illustrated in FIG. 5(b), the compressed air feed pipe supporting bracket 54 has formed therein two holding holes (first holding hole 54a and second holding hole 54b) for holding the joint portions between the compressed air feed pipes 102 (FIG. 2) and the compressed air feed pipes 28.

The production apparatus 100 may not have the support stand 50, and the cooling unit 20 in this case may, for example, be fixed to the cabinet 40. In this case, the cooling unit 20 is preferably fixed to the cabinet 40, so as to vary the fixing position of the cooling unit 20 to the cabinet 40 (height position, position in the width direction of the cabinet 40, etc.), and posture of the cooling unit 20 fixed to the cabinet 40.

The cooling unit 20 may not have the flexible tube 22, the L-shaped joint 24, the outlet pipe 25 and so forth. In this case, the vortex tube 21 may be disposed so that the cold air may be blown out from the cold air output unit 21b of the vortex tube 21 directly towards the solid phase extraction unit 10.

In this embodiment, the production apparatus 100 may be designed to have a cover 70 that covers the solid phase extraction unit 10. In this case, the cold air blower is designed to feed cold air inside the cover 70.

By feeding cold air inside the cover 70 that covers the solid phase extraction unit 10, it becomes possible to feed cold air towards the solid phase extraction unit 10 while being rectified by the cover 70. Therefore, cooling efficiency of the solid phase extraction unit 10 may be improved.

The cover 70 is provided to the tip of the outlet pipe 25, so as to protrude from the outlet pipe 25 towards the tip direction thereof. As illustrated in FIG. 5 (a) and FIG. 6(b), the cover 70 is formed to have a C-shape cross section or U-shape cross section, opened towards the tip (distal side as viewed from the outlet pipe 25). In one example, the cover 70 is formed so that the opening width thereof widens in the direction departing from the outlet pipe 25 (FIG. 6(b)).

The cover 70, for example, has a first wall portion 71 that is provided at the end of the outlet pipe 25 in a state perpendicular to the outlet pipe 25, and a pair of second wall portions 72 that are arranged to intersect with the first wall portion 71 and diagonally opposed to each other. The first wall portion 71 has an opening 71a formed so as to communicate with the outlet port 25a, allowing the cold air blown out from the outlet port 25a to be fed through the opening 71a to the inside of the cover 70.

The outlet pipe 25 is arranged so that the solid phase extraction unit 10 lies on an extension line of the outlet pipe 25, and so that an axial direction of the outlet pipe 25 intersects (orthogonal, for example) an axial direction of the solid phase extraction unit 10.

The cover 70 may cover the entire portion of the solid phase extraction unit 10, or may cover a part of the solid phase extraction unit 10. In the example illustrated in FIG. 6(a), a half portion of a small diameter portion 10b (described later) of the solid phase extraction unit 10, which resides closer to the outlet pipe 25, is covered by the cover 70.

The cover 70 may be made of an unspecified material, and is preferably made, for example, of a material (resin, etc.) having a thermal conductivity smaller than that of the heat sink 80 described later.

The production apparatus 100 does, however, not always necessarily have the cover 70.

As illustrated in FIG. 6 (a) and FIG. 6 (b), the production apparatus 100, for example, has a heat sink 80 disposed around the solid phase extraction unit 10.

A cylindrical metal component, for example, may be used as the heat sink 80, and solid phase extraction unit 10 may be disposed so as to be inserted in the heat sink 80. The heat sink 80 may be composed of an unspecified material, but is preferably made of a material with high thermal conductivity, which is exemplified by aluminum, copper, or alloy of them.

The cold air output from the outlet port 25a is blasted against the outer surface of the heat sink 80. The solid phase extraction unit 10 is thus cooled while mediated by the heat sink 80.

Being cooled while mediated by the heat sink 80, the solid phase extraction unit 10 may be cooled more uniformly.

The solid phase extraction unit 10 is, for example, what is referred to as "short column", and has a large diameter portion 10a and a small diameter portion 10b, both being formed into columnar shape, and a male connector 11 and a female connector 12, all being arranged coaxially. In this case, for example, the small diameter portion 10b of the solid phase extraction unit 10 may inserted to the heat sink 80. The cooling unit 20 is therefore designed to cool the small diameter portion 10b locally. The cooling unit 20 may, however, be designed to uniformly cool the entire portion of the solid phase extraction unit 10.

In a portion of the solid phase extraction unit 10 excluding the male connector 11 and the female connector 12, that is, in the large diameter portion 10a and the small diameter portion 10b, a packing material is packet. It is therefore allowable to locally cool the large diameter portion 10a and small diameter portion 10b, using the cooling unit 20.

For the case where the solid phase extraction unit 10 is arranged with the axial direction thereof aligned horizontally as shown in FIG. 6(a), the solid phase extraction unit 10 can support the heat sink 80 without causing dropping of the heat sink 80 from the solid phase extraction unit 10, even if the heat sink 80 is not always necessarily fixed to the solid phase extraction unit 10. It is, however, also preferable to fix the heat sink 80 to the solid phase extraction unit 10.

The cover 70, when owned by the production apparatus 100, may be designed to cover the solid phase extraction unit 10, while placing for example the heat sink 80 in between.

Note that, in FIG. 6(b), the outlet pipe 25, the cover 70 and the heat sink 80 are given by the cross sections, and the other components are given by the side geometries. In FIG. 6(b), the solid phase extraction unit 10 is not illustrated.

As illustrated in FIG. 7, the production apparatus 100 has a temperature detection unit 90 that detects the surface temperature of the solid phase extraction unit 10, a control unit 110 that takes part in operational control of the vortex tube 21 in response to the result of detection given by the temperature detection unit 90, and a solenoid valve 120 controlled by the control unit 110.

As illustrated in FIG. 6(a), the temperature detection unit 90 is, for example, a thermocouple, has a terminal 91 provided at one end thereof, and the terminal 91 is fixed to the heat sink 80 using a fixing member 92 such as a bolt. That is, the temperature detection unit 90 indirectly detects the surface temperature of the solid phase extraction unit 10, for example, by detecting the temperature (surface temperature) of the heat sink 80. Note that the temperature detection unit 90 may directly detect the surface temperature of the solid phase extraction unit 10.

The solenoid valve 120 is, for example, provided to the compressed air feed pipe 102 (FIG. 2), and switches between the states of the vortex tube 21 fed with compressed air, and not fed with compressed air, while controlled by the control unit 110.

The control unit 110, for example, brings the solenoid valve 120 under feedback control in response to the result of detection given by the temperature detection unit 90, so that the surface temperature of the solid phase extraction unit 10 may be kept within a predetermined range.

For instance, the solenoid valve 120 is controlled so as to feed compressed air to the vortex tube 21 until the temperature detected by the temperature detection unit 90 decreases down to, or below a target temperature having been arbitrarily set in advance, and so as to stop feeding the compressed air to the vortex tube 21 when the temperature detected by the temperature detection unit 90 decreases down to, or below the target temperature. In the embodiments described hereinbelow, this type of feedback control will be referred to as "first control".

Note, however, that a more precise feedback control such as PID (Proportional Integral Derivative) control may also be employed to control operations of the solenoid valve 120. In the embodiments described hereinbelow, the PID control will be referred to as "second control".

Besides the cooling unit 20, the production apparatus 100 may have a temperature conditioning unit that keeps the whole production apparatus 100 conditioned (or cooled) at a constant room temperature. The cooling unit 20 in this design can cool the solid phase extraction unit 10 to a temperature below the cooling temperature by using the temperature conditioning unit.

### <Modified Example of Second Embodiment>

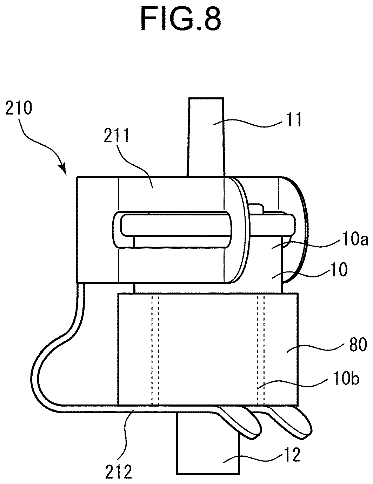

FIG. 8 is a front elevation illustrating the solid phase extraction unit 10 and the heat sink 80 of the apparatus for producing a radiolabeled compound according to a modified example of the second embodiment (not entirely illustrated).

In this modified example, the solid phase extraction unit 10 is disposed with the axial direction thereof laid in the direction crossed at an angle (perpendicular direction, for example) with the horizontal direction, with the small diameter portion 10b thereof disposed below the large diameter portion 10a, and with the small diameter portion 10b inserted in the heat sink 80. In this case, the heat sink 80 is preferably fixed to the solid phase extraction unit 10 using a clamp 210, in order to suppress the heat sink 80 from dropping from the small diameter portion 10b.

The clamp 210, for example, has a first support part 211 that supports the large diameter portion 10a at the outer circumference thereof, and a second support part 212 that supports a metal cylinder composing the heat sink 80, at the lower end of the metal cylinder. The second support part 212 has a pair of supporting pieces that engage with the lower end of the metal cylinder composing the heat sink 80, and the pair of supporting pieces are disposed on both sides of the female connector 12 while placing the female connector 12 in between.

The heat sink 80 has an inner diameter larger than the outer diameter of the small diameter portion 10b, but smaller than the outer diameter of the large diameter portion 10a. The pair of supporting pieces of the second support part 212 are composed of an elastically deformable material, and energize the heat sink 80 by the spring action against the large diameter portion 10a (that is, upwardly energize) . In this way, the heat sink 80 may be fixed stably to the solid phase extraction unit 10 by the clamp 210.

For example, the whole part of the clamp 210 is composed of a metal. The clamp 210 may, however, be composed of an elastically deformable resin material.

### [Third Embodiment]

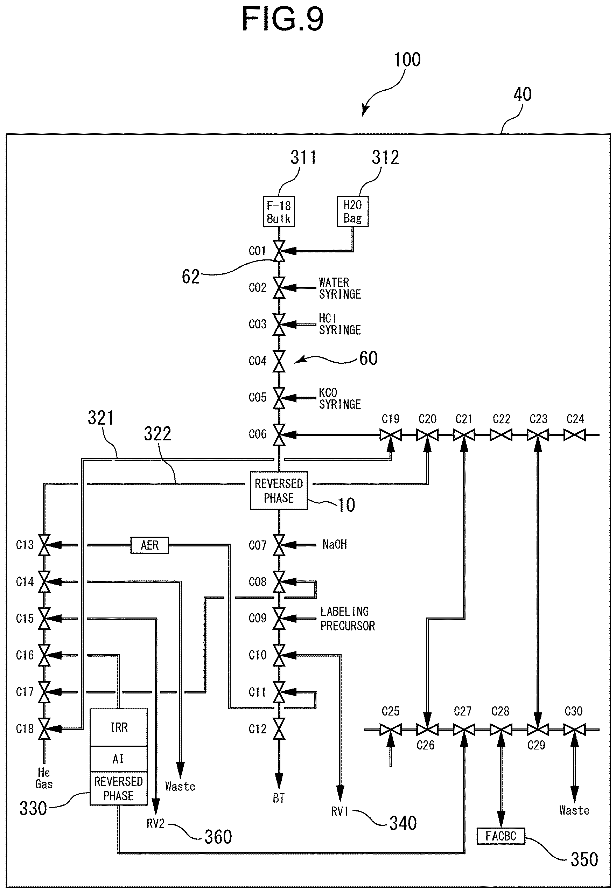

FIG. 9 is a drawing for explaining the apparatus for producing a radiolabeled compound 100 according to the third embodiment. The production apparatus 100 of this embodiment is different from the above-described production apparatus 100 of the second embodiment in the aspects below, but is same as the production apparatus 100 of the second embodiment in the other aspects.

The production apparatus 100 of this embodiment is suitably designed so as to be functionalized as described below referring to FIG. 9.

To the valve holder 32 (see FIG. 2) of the production apparatus 100, there is attached the three-way cocks 62 of the channel cartridge 60 such as those exemplified in FIG. 3. The solid phase extraction unit 10 may be disposed, as illustrated in FIG. 9, between the three-way cocks 62 of the channel cartridge 60, so as to form a vertical in-line arrangement of such plurality of three-way cocks 62 and the solid phase extraction unit 10. The solid phase extraction unit 10 in this design is disposed with the axial direction thereof aligned perpendicularly, unlike the arrangement illustrated in FIG. 2.

In FIG. 9, the individual three-way cocks 62 are denoted by corresponding symbols C01 to C30, given on the left side, the upper side, or the lower side thereof.

Among them, the three-way cock C01 has connected thereto a tank 311 that contains ¹⁸F ion-containing water-¹⁸O, and a tank 312 that contains water, the three-way cock C02 has attached thereto a syringe filled with water, and the three-way cock C03 has attached thereto a syringe filled with hydrochloric acid. The tanks 311 and 312 may be mounted on the cabinet 40. The three-way cocks C19 and C18 are connected via a tube 321, and the three-way cocks C20 and C13 are connected via a tube 322. The three-way cocks C06 and C07 are connected while placing a solid phase extraction unit 10 that is a reversed phase column called tC18 column therebetween. Also in this embodiment, the solid phase extraction unit 10 is a short column.

The solid phase extraction unit 10 has a vessel made of resin, and a packing material packet in the vessel. The packing material is silica gel and has, for example, a structure in which an alkyl chain having 1 to 18 carbon atoms is bonded to a support via silicon. More specifically, the packing material is a chemically bonded porous spherical silica gel bead having the surface modified, for example, with octadecylsilyl (C₁₈H₃₇Si) group, and this packing material is packet as a stationary phase into the solid phase extraction unit 10.

An exemplary production of [¹⁸F]FACBC, using the production apparatus 100 illustrated in FIG. 9, will be explained.

### <Fluorination Process>

First, the handles of the three-way cocks C06, C20 and C13 are turned to allow the ¹⁸F ion-containing water-¹⁸O to pass through an anion exchange resin (AER) such as QMA, to thereby allow ¹⁸F ion to adhere to the anion exchange resin. Water-¹⁸O is allowed to pass through the three-way cock C12, and collected into a collection bottle (BT). The collection bottle (BT) is, for example, mounted on the cabinet 40.

Radioactivity of ¹⁸F ion collected by the anion exchange resin such as QMA is detectable, for example, by a radiation detector built in the cabinet 40.

Next, the handles of the three-way cocks C05, C013, C11 and C10 are respectively turned to activate the syringe drive mechanism attached to the cabinet 40, so as to eject an aqueous potassium carbonate solution to thereby elute ¹⁸F ion from the anion exchange resin, and the eluate is collected through the three-way cock C10 into a first reaction vessel (RV1). Also the first reaction vessel (RV1) may be mounted to the cabinet 40.

An acetonitrile solution of Kryptofix 222 (product name) is added to the first reaction vessel (RV1), and the handles of the three-way cocks C18, C17, C08, C10 are respectively turned to azeotropically dry the mixture under flow of an inert (He) gas.

Note that the first reaction vessel (RV1) has attached thereto lines for connecting a suction pump for vent, and for connecting a collection bottle for collecting evaporated acetonitrile and water, while placing change-over valves in between. The lines are not illustrated. The handle of the three-way cock C09 is then turned to activate the syringe drive mechanism to thereby add an acetonitrile solution, which is a labeling precursor compound of [¹⁸F]FACBC, through the three-way cock C10 to the first reaction vessel (RV1), to thereby a [¹⁸F] fluorination reaction is carried out.

By introducing ¹⁸F as a radioisotope into the labeling precursor compound in this way, the [¹⁸F] fluorinated compound may be produced as an intermediate compound.

Here, the first reaction vessel (RV1) functions as a label introducing unit 340 which introduces the radioisotope into the labeling precursor compound.

### <Column Collection Process>

After completion of the reaction, the handles of the three-way cocks C06, C13, C14 are respectively turned to allow the solid phase extraction unit 10, which is a reversed phase column, to adsorb the [¹⁸F] fluorinated compound. Acetonitrile used as a solvent is collected through the three-way cock C14 into a waste vial (Waste).

### <Alkaline Hydrolysis Process (First NaOH Process, Second NaOH Process)>

The handles of the three-way cocks C07, C15 are then respectively turned to activate the syringe drive mechanism, so as to allow the aqueous sodium hydroxide solution filled in the syringe to pass in two portions through the solid phase extraction unit 10, which is the revered phase column, for alkali hydrolysis process. In this way, the deprotection (de-esterification) of the protective group of the carboxylic acid of the intermediate compound ([¹⁸F] fluorinated compound) on the solid phase extraction unit 10 is carried out.

That is, as the specific process, de-esterification by alkali hydrolysis is carried out in the solid phase extraction unit 10.

In this specification, the alkali hydrolysis by the first passage of the aqueous sodium hydroxide solution may occasionally be referred to as "first NaOH process", and the alkali hydrolysis by the first passage of the aqueous sodium hydroxide solution may occasionally be referred to as "second NaOH process".

The aqueous sodium hydroxide solution after used for the alkali hydrolysis process is collected in a second reaction vessel (RV2). Here, the second reaction vessel (RV2) may be mounted on the cabinet 40. The second reaction vessel (RV2) has attached thereto a line for connecting a vent while placing change-over valves in between. The line is not illustrated.

### <Water Rinsing Process>

Next, the handles of the three-way cocks C01, C02 are respectively turned to activate the syringe drive mechanism, to thereby fill water in the tank 312 into the syringe attached to the three-way cock C02. The handles of the three-way cocks C02, C15 are then respectively turned to activate the syringe drive mechanism, so as to inject water into the solid phase extraction unit 10, which is a reversed phase column, to thereby elute the de-esterified intermediate compound ([¹⁸F] fluorinated compound) out from the solid phase extraction unit 10, and the eluate is collected in the second reaction vessel (RV2). The de-esterified intermediate compound is mixed, in the second reaction vessel (RV2), with the previously collected aqueous sodium hydroxide solution.

### <Acid Hydrolysis Reaction Process>

Next, the handles of the three-way cock C03 is turned to activate the syringe drive mechanism, so as to add hydrochloric acid contained in the syringe to the second reaction vessel (RV2). Deprotection of the amino protective group of the de-esterified intermediate compound and deprotection of the carboxylic acid protective group are carried out by performing a hydrolysis reaction (acid hydrolysis reaction process) under an acidic condition in the second reaction vessel (RV2). Here, the second reaction vessel (RV2) functions as an acid hydrolyzing unit 360.

### <Purification Process>

The handles of the three-way cocks C15, C016, C27, C28 are then respectively turned, so as to allow the liquid to pass through an ion retardation resin (IRR), alumina (Al) and a reversed phase column (referred to as "purification column 330", hereinafter), to thereby collect [¹⁸F]FACBC into the intended product collection vessel 350.

The handles of the three-way cocks C01, C02 may optionally be turned to activate the syringe drive mechanism, so as to fill water in the tank 312 into the syringe attached to the three-way cock C02, and the handles of the three-way cocks C02, C06, C19, C18, C16 may respectively be turned to activate the syringe drive mechanism, to thereby rinse the purification column 330 with water.

After the liquid is allowed to pass through the purification column 330, and before [¹⁸F]FACBC is collected through the three-way cock C28 into the intended product collection vessel 350, the handle of the three-way cock C27 may be turned to inject the eluate into an HPLC (high performance liquid chromatography) column (not illustrated) to purify [¹⁸F]FACBC by HPLC. In this case, a pump is preliminarily activated to fill the HPLC column with a developing solvent. The developing solvent that passed through the HPLC column is discarded through the three-way cocks C24, C23, C29 and C30. After injecting [¹⁸F]FACBC from an injector into the HPLC column, a peak of [¹⁸F]FACBC is identified using a radiation detector, and the peak of [¹⁸F]FACBC is collected through the three-way cock C28, by operating the handles of the three-way cocks C28, C29.

In this way, [¹⁸F]FACBC may be obtained.

Here, the first reaction vessel (RV1), in which fluorination process ([¹⁸F] fluorination reaction) is carried out, functions as a label introducing unit 340 that introduces radioactive fluorine (¹⁸F) as a radioisotope into the labeling precursor compound. The process for producing the radioactive fluorine labeled ester, which is an intermediate compound, by introducing a radioisotope into the labeling precursor compound in the first reaction vessel (RV1) (label introducing process) is performed at a temperature higher than room temperature.

Meanwhile, the process of reacting with the intermediate compound (radioactive fluorine labeled ester), in the solid phase extraction unit 10 (specific process) is performed while cooling the solid phase extraction unit 10 by the cooling unit 20.

As described above, the production apparatus 100 of this embodiment further has the label introducing unit 340 (first reaction vessel (RV1)) that introduces a radioisotope into the labeling precursor compound, wherein the production apparatus 100 carries out label introducing process that introduces the radioisotope into the labeling precursor compound, in the label introducing unit 340 at a temperature higher than room temperature, to thereby produce the intermediate compound; and the specific process which is reaction of the intermediate compound obtained by the label introducing process.

In this way, it can be suppressed that the troubles are caused by the temperature of the solid phase extraction unit 10 becoming high during the specific process.

Since the label introducing process is performed at a temperature higher than room temperature, so that it is not preferable to cool the whole production apparatus 100 down to a certain constant temperature using the cooling unit 20. It is instead preferable to locally cool the solid phase extraction unit 10 using the cooling unit 20.

Here, when [¹⁸F]FACBC is purified by HPLC by using the HPLC column as descried above, the HPLC column need not be cooled using the cooling unit 20. That is, when using the production apparatus 100 having both of the solid phase extraction unit 10 and the HPLC column, the cooling unit 20 selectively cools the solid phase extraction unit 10, out of the solid phase extraction unit 10 and the HPLC column.

The specific process is a hydrolysis reaction of the ester group performed in the presence of alkali for the intermediate compound ([¹⁸F] fluorinated compound) having an ester group.

That is, the specific process is an alkali hydrolysis that de-esterifies the intermediate compound using an aqueous alkaline solution.

The aqueous alkaline solution employable for de-esterification is, for example, aqueous sodium hydroxide solution, or, aqueous potassium hydroxide solution.

The production apparatus 100 of this embodiment further has an acid hydrolyzing unit 360 in which the hydrolysis reaction is carried out under the acidic condition, of the compound obtained by the reaction in the specific process.

In this embodiment, the acid hydrolyzing unit 360 is the second reaction vessel (RV2), and the hydrolysis reaction is the above-described acid hydrolysis reaction process. In the acid hydrolysis reaction process, not only deprotection of the amino protective group, but also deprotection of the carboxylic acid protective group is carried out.

More specifically, in the specific process, a compound represented by the formula (2) below is obtained by holding the intermediate compound represented by the formula (1) below in the solid phase extraction unit 10 and passing the alkaline solution through the solid phase extraction unit 10 while cooling the solid phase extraction unit 10 by the cooling unit 20: 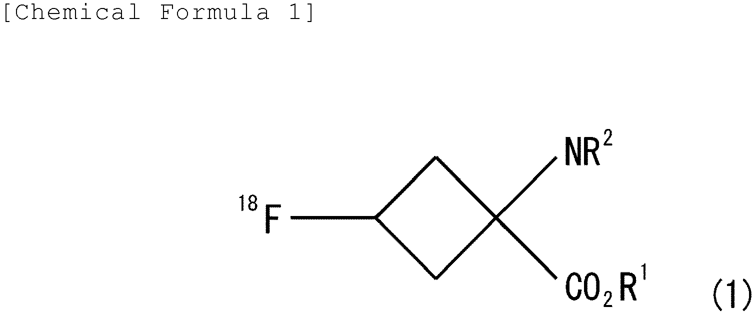 (in the formula, R¹ represents a straight-chain or branched alkyl chain having 1 to 10 carbon atoms or an aromatic substituent, and R² represents a protective group) 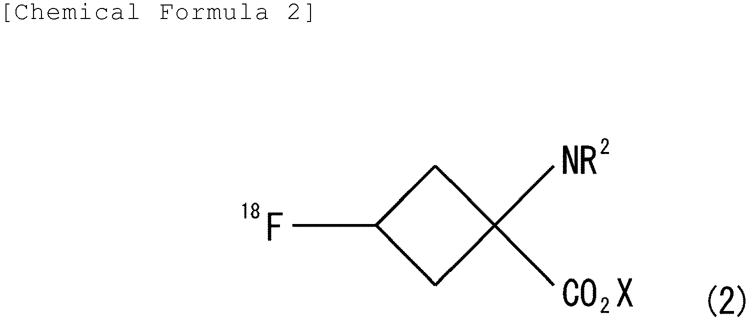 (in the formula, X represents a cation (for example, sodium or potassium) contained in the alkaline solution used in the de-esterification, and R² represents a protective group).

In the hydrolysis reaction under an acidic condition (the above-mentioned acid hydrolysis reaction process), the deprotection of the amino protective group (and the deprotection of the carboxylic acid protective group) is carried out for the compound obtained by the specific process, by carrying out the hydrolysis reaction under the acidic condition, to obtain a compound represented by the formula (3) below.

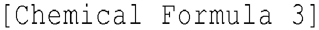

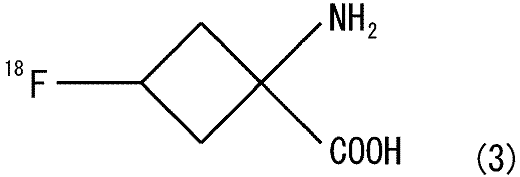

The period over which the solid phase extraction unit 10 is cooled by the cooling unit 20 is not specifically limited, so long as it contains at least a part of the period of the alkali hydrolysis process (specific process). Note, however, that the period preferably contains the whole period of the alkali hydrolysis process (specific process), which may range from the halfway or after the finish time of fluorination process (label introducing process), up to the halfway or before the start time of acid hydrolysis reaction process, and even may range from the time after the finish time of fluorination process up to the time before the start time of water rinsing process (that is, only within the period of specific process).

Here, as described above, if the temperature of the solid phase extraction unit 10 becomes high during the deprotection process in the process of producing [¹⁸F]FACBC, the purification column 330 (the ion retardation resin (IRR), alumina (Al) and reversed phase columns) may be clogged and become unable to feed the reaction liquid through the purification column 330, making the purification of [¹⁸F]FACBC difficult.

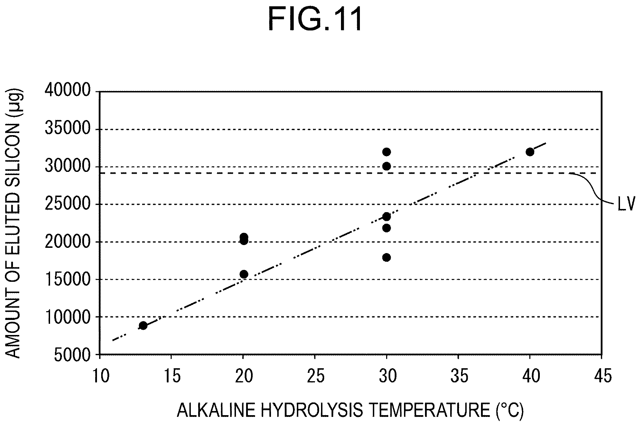

This will be explained referring to FIG. 11.

FIG. 11 is a drawing illustrating a relation between the surface temperature of the solid phase extraction unit 10, and the amount of eluted silicon observed on the downstream side of the solid phase extraction unit 10, in the process of producing [¹⁸F]FACBC. More specifically, the drawing illustrates a relation between the surface temperature of the solid phase extraction unit 10 and the amount of eluted silicon on the downstream side of the solid phase extraction unit 10, during the alkali hydrolysis process (the first NaOH process and the second NaOH process).

Note that, in the example shown in FIG. 11, the surface temperature of the solid phase extraction unit 10 was indirectly detected by detecting the surface temperature of the heat sink 80.

It is understood from FIG. 11 that there is a positive correlation between the amount of eluted silicon and the surface temperature of the solid phase extraction unit 10 during alkali hydrolysis process.

The present inventors presumed that silicon detected on the downstream side of the solid phase extraction unit 10 was eluted from the solid phase extraction unit 10. More specifically, the silicon eluted from the solid phase extraction unit 10 was considered to be derived from the packing material of the solid phase extraction unit 10. That is, it was considered that the octadecylsilyl group (C₁₈H₃₇Si) was separated from the chemically bonded porous spherical silica gel bead and eluted from the solid phase extraction unit 10.

It was thought that the reaction liquid could not be delivered in the purification column, due to clogging of the purification column with silicon eluted from the solid phase extraction unit 10.

As a result of further investigations by the inventors of the present invention, it was found that, by performing the alkali hydrolysis process while keeping the surface temperature of the solid phase extraction unit 10 at 30°C or below, the occurrence frequency of clogging of the purification column 330 can be reduced, and, by performing the alkali hydrolysis process while keeping the surface temperature of the solid phase extraction unit 10 at 20°C or below, the occurrence frequency of clogging of the purification column 330 can be further reduced.

On the other hand, it was found that the occurrence frequency with which the level of the elution amount of silicon increases up to the level of clogging of the purification column 330 is likely to occur (up to the level beyond acceptable level LV in FIG. 11), when the alkali hydrolysis process is performed with the surface temperature of the solid phase extraction unit 10 set at a higher temperature than 30°C. More specifically, for example, when the alkali hydrolysis process is performed with the surface temperature of the solid phase extraction unit 10 set at 40°C, the amount of eluted silicon was found to more frequently increase up to a level causing clogging of the purification column 330, as compared with the case at 30°C.

Hence, clogging of the reaction liquid in the purification column may more suitably be suppressed during the alkali hydrolysis process, by keeping the surface temperature of the solid phase extraction unit 10 at 30° or below. The solid phase extraction unit 10 during the alkali hydrolysis process is more preferably kept at 25°C or below.

That is, during the specific process, it is preferable that the hydrolysis in the presence of alkali for the ester group of the intermediate compound is carried out while keeping the surface temperature of the solid phase extraction unit 10 at 30°C or below. It is more preferable that the hydrolysis is carried out while keeping the surface temperature of the solid phase extraction unit 10 at 25°C or below.

Too low temperature of the solid phase extraction unit 10 degrades efficiency of the alkali hydrolysis process, therefore, the surface temperature of the solid phase extraction unit 10 during the alkali hydrolysis is preferably kept at 15°C or above. In this way, production time of a radiolabeled compound containing nuclide with a short half-life may be suppressed from becoming time-consuming.

Summarizing the above, the surface temperature of the solid phase extraction unit 10 during the alkali hydrolysis is preferably set to 15°C or above and 25°C or below.

In this embodiment, as described above, the alkali hydrolysis process is carried out while cooling the solid phase extraction unit 10 by the cooling unit 20.

Hence, as explained below, it can be suppressed that the temperature of the solid phase extraction unit 10 becomes high. As a consequence, the amount of eluted silicon from the solid phase extraction unit 10 during the alkali hydrolysis process may be kept at a low level, and thereby the clogging of the reaction liquid in the purification column can be suppressed.

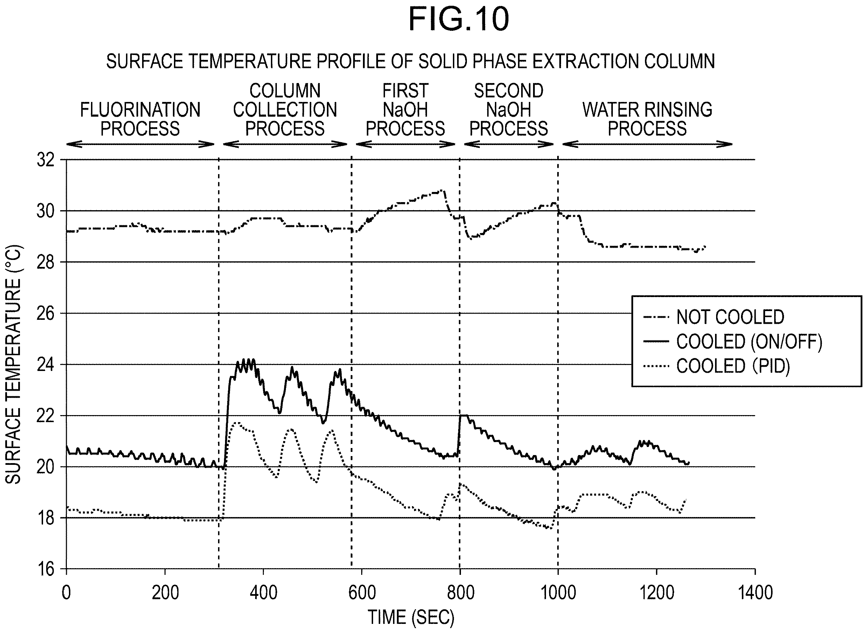

FIG. 10 is a time chart illustrating exemplary temperature changes of the solid phase extraction unit 10 of the apparatus for producing a radiolabeled compound 100 according to the third embodiment, and of the solid phase extraction unit of the apparatus for producing a radiolabeled compound according to a comparative embodiment, based on actually measured results.

FIG. 10 shows changes of the surface temperature of the heat sink 80, that is, the temperature detected by the temperature detection unit 90 in the period (referred to as "measurement period", hereinafter) in which the above-described fluorination process, the column collection process, alkali hydrolysis process (the first NaOH process, the second NaOH process) and water rinsing process is carried out.

Here, FIG. 10 shows as a preferred example of this embodiment, in which the solid phase extraction unit 10 was cooled respectively by two types of feedback control - the first control (denoted by "COOLED (ON/OFF)" in the drawing), and the second control (denoted by "COOLED (PID)" in the drawing) respectively mentioned above. In both of the first control and the second control, a target temperature of cooling was 20°C.

On the other hand, the apparatus for producing a radiolabeled compound according to the comparative embodiment is different from the production apparatus 100 of this embodiment, in that it does not have the cooling unit 20. Hence, in an example of using the apparatus for producing a radiolabeled compound of the comparative embodiment (denoted by "NOT COOLED" in the drawing), the solid phase extraction unit 10 was not cooled by the cooling unit 20 in the measurement period.

As shown in FIG. 10, in the comparative embodiment, an elevation of the surface temperature of the heat sink 80 was observed in both of the first NaOH process and the second NaOH process, and there was a timing at which the surface temperature of the heat sink 80 was 30°C or higher in both of the first NaOH process and the second NaOH process.

In contrast, in this embodiment, the surface temperature of the heat sink 80 was kept at 25°C or below and 15°C or above over the entire measurement period (including the first NaOH process and the second NaOH process), both by the first control and the second control.

As described above, according to this embodiment, it can be suppressed that the temperature of the solid phase extraction unit 10 becomes high during the specific process, therefore, troubles (clogging of the purification column) possibly caused by the temperature of the solid phase extraction unit 10 becomes high during the specific process may be suppressed from occurring.

Although, the third embodiment has explained the case where the production apparatus 100, described in the second embodiment, was used to produce [¹⁸F]FACBC, the production apparatus 100 described in the second embodiment may alternatively be used for producing other radiolabeled compound such as [¹⁸F]flutemetamol and 2-[¹⁸F]fluoro-2-deoxy-D-glucose (FDG), by suitable modifications, such as, selecting and arranging the constituents, changing the operating modes of the three-way cocks and the valve, and so on.

Among them, FDG may be produced as described below.

First, a radioisotope is introduced into a labeling precursor compound for producing FDG in the same way as in the production of [¹⁸F]FACBC, to thereby produce an intermediate compound. The intermediate compound is 2-[¹⁸F]fluoro-1,3,4,6-tetra-O-acetyl-D-glucose, which is abbreviated as tetraacetylfluoroglucose or TAFg.

Next, the intermediate compound (TAFg) is adsorbed to the solid phase extraction unit 10, and pass an alkaline solution such as NaOH solution through the solid phase extraction unit 10 once, or twice or more times, thereby, a deprotection process (alkali hydrolysis process) of the intermediate compound (TAFg) is carried out. Thereafter, the water rinsing process, the acid hydrolysis reaction process and the purification process are performed in the same manner as in the production of [¹⁸F]FACBC, thereby, FDG can be obtained.

Here, in the production of FDG, the radioisotope is introduced into the labeling precursor compound in a vessel (label introducing unit) which is different from the solid phase extraction unit 10, at a temperature higher than room temperature.

The deprotection process (alkali hydrolysis process) performed on the intermediate compound (TAFg), having been obtained by introducing the radioisotope into the labeling precursor compound, is performed in the solid phase extraction unit 10 while cooling the solid phase extraction unit 10 by the cooling unit 20.

In the production of [¹⁸F] flutemetamol, the deprotection for the intermediate compound in which the radioisotope is introduced may be carried out by using an acid such as hydrochloric acid. The deprotection may be carried out in the solid phase extraction unit 10 in the state that the intermediate compound is adsorbed thereto, while locally cooling the solid phase extraction unit 10.

In this case, the production apparatus 100 may perform the deprotection, respectively in a plurality of (two of, for example) solid phase extraction units 10. In this case, the production apparatus 100 may have two cooling units 20 provided in one to one correspondence to the individual solid phase extraction units, making each cooling unit 20 cool the corresponding solid phase extraction unit 10. Furthermore, in this case, the support stand 50 may have, for example, two cooling units 20 supported thereon.

The specific process performed for the intermediate compound obtained in the label introducing process is not limited to the reaction of the intermediate compound, but may also be a purification of an intermediate compound.

The hydrolysis reaction, performed in the acid hydrolysis unit under an acidic condition, is not limited to a hydrolysis reaction for a compound obtained by performing the reaction of the intermediate compound in the specific process, but also may be a hydrolysis reaction for a compound obtained by performing a purification of the intermediate compound in the specific process.

### <Modified Example>

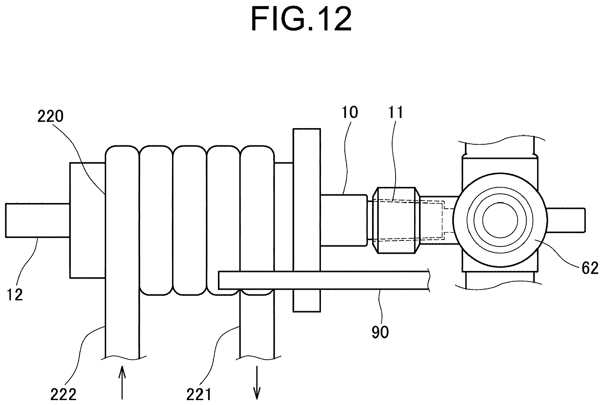

Although the second embodiment have detailed an exemplary case where the cooling unit 20 cools the solid phase extraction unit 10 with cold air, the cooling unit may employ a system that cools the solid phase extraction unit 10 with circulating water (water cooling system) as described above. Here, an example of the configuration of the cooling unit of a water cooling type will be described with reference to FIG. 12.

In this case, as illustrated in FIG. 12, the cooling unit has a cooling pipe 220 made of metal. The cooling pipe 220 is preferably a copper pipe, from the viewpoint of thermal conductivity. The cooling pipe 220 has a winding portion wound around the solid phase extraction unit 10. The winding portion is formed, for example, by winding a part of the cooling pipe 220 a plurality of times with tight winding.

A portion of the solid phase extraction unit 10 around which the cooling pipe 220 is wound (that is, a portion provided with the winding portion) is, for example, a cylindrical main body (corresponded to the above-described large diameter portion 10a and the small diameter portion 10b).

The cooling pipe 220 has, attached to the surface thereof, a thermocouple as the temperature detection unit 90.

The winding portion of the cooling pipe 220 has at one end thereof an inlet end 222 through which circulating water (cooling water) is fed into the winding portion, and has at the other end thereof an outlet end 221 through which the circulating water is discharged out from the winding portion.

The solid phase extraction unit 10 allows for input of a fluid such as chemical liquid from the right side in FIG. 12 (from the side of three-way cock 62), and allows for output of the fluid such as chemical liquid to the left side in FIG. 12. Therefore, the temperature of the solid phase extraction unit 10 is higher in the right side of FIG. 12. The outlet end 221 of the winding portion of the cooling pipe 220 is positioned on the right side of the inlet end 222. In other words, a portion where the circulating water flowing through the winding portion of the cooling pipe 220 reaches the highest temperature is positioned on the high temperature side of the solid phase extraction unit 10 (on the right side in FIG. 12). This easily creates a large temperature difference between the circulating water and the solid phase extraction unit 10, therefore, cooling of the solid phase extraction unit 10 with the circulating water is to be stabilized.

This embodiment encompasses the technical ideas below.
(1) An apparatus for producing a radiolabeled compound which produces a radiolabeled compound by introducing a radioisotope into a non-radioactive labeling precursor compound, the apparatus including:
   a solid phase extraction unit in which a specific process which is a reaction of an intermediate compound, a purification of the intermediate compound, or a purification of the radiolabeled compound is carried out; and
   a cooling unit that cools the solid phase extraction unit, when the specific process is carried out.
(2) The apparatus for producing a radiolabeled compound according to (1), wherein the solid phase extraction unit has a solid phase carrier to which a silyl group is bonded, the specific process is carried out in the presence of alkali.
(3) The apparatus for producing a radiolabeled compound according to (1) or (2), further including a label introducing unit that introduces the radioisotope into the labeling precursor compound, and
   the production apparatus carries out:
   label introducing process that introduces the radioisotope into the labeling precursor compound, in the label introducing unit at a temperature higher than room temperature, to thereby produce the intermediate compound; and
   the specific process which is the reaction or the purification of the intermediate compound obtained by the label introducing process.
(4) The apparatus for producing a radiolabeled compound according to (3), wherein the specific process is a hydrolysis reaction of the ester group performed in the presence of alkali for the intermediate compound having an ester group.
(5) The apparatus for producing a radiolabeled compound according to (3) or (4), further including an acid hydrolyzing unit in which an acid hydrolysis reaction of the compound, obtained by the reaction or the purification conducted as the specific process, is carried out under an acidic condition.
(6) The apparatus for producing a radiolabeled compound according to any one of (1) to (5), wherein in the specific process,
   a compound represented by the formula (2) above is obtained by holding the intermediate compound represented by the formula (1) above in the solid phase extraction unit and passing the alkaline solution through the solid phase extraction unit while cooling the solid phase extraction unit by the cooling unit.
(7) The apparatus for producing a radiolabeled compound according to any one of (1) to (6), wherein the cooling unit contains a cold air blower that cools the solid phase extraction unit with cold air.
(8) The apparatus for producing a radiolabeled compound according to (7), wherein the cold air blower includes a vortex tube having an introduction unit that introduces therein compressed air, a cold air output unit that blows out the cold air, and a hot air output unit that blows out hot air,
   the vortex tube being disposed so that the hot air output unit blows out the hot air towards the direction opposite to the solid phase extraction unit with reference to the introduction unit.
(9) The apparatus for producing a radiolabeled compound according to (7) or (8), further including a cover that covers the solid phase extraction unit, and
   the cold air blower supplies the cold air inside the cover.
(10) The apparatus for producing a radiolabeled compound according to any one of (1) to (9), further including a heat sink disposed around the solid phase extraction unit.
(11) A method for producing a radiolabeled compound for producing a radiolabeled compound by introducing a radioisotope into a non-radioactive labeling precursor compound, the method including:
   performing a specific process in a solid phase extraction unit holding an intermediate compound or the radiolabeled compound retained therein, while locally cooling the solid phase extraction unit, the specific process being any one of a reaction of the intermediate compound, a purification of the intermediate compound, or a purification of the radiolabeled compound.
(12) The method for producing a radiolabeled compound according to (11), wherein the solid phase extraction unit includes a solid phase carrier to which a silyl group is bonded, and the specific process is performed in the presence of alkali.
(13) The method for producing a radiolabeled compound according to (11) or (12), wherein, in the specific process, a hydrolysis is performed in the presence of alkali for an ester group of the intermediate compound having the ester group, with the surface temperature of the solid phase extraction unit kept at 30°C or below.

This application is based on Japanese Patent Application No. 2015-115179, filed on June 5, 2015, the entire content of which is incorporated hereinto by reference.

## Claims

1. An apparatus for producing a radiolabeled compound which produces a radiolabeled compound by introducing a radioisotope into a non-radioactive labeling precursor compound, the apparatus comprising:
a solid phase extraction unit in which a specific process which is a reaction of an intermediate compound, a purification of the intermediate compound, or a purification of the radiolabeled compound is carried out; and
a cooling unit that cools the solid phase extraction unit, when the specific process is carried out.

2. The apparatus for producing a radiolabeled compound according to Claim 1, wherein the solid phase extraction unit has a solid phase carrier to which a silyl group is bonded, the specific process is carried out in the presence of alkali.

3. The apparatus for producing a radiolabeled compound according to Claim 1 or 2, further comprising a label introducing unit that introduces the radioisotope into the labeling precursor compound, and
the production apparatus carries out:
label introducing process that introduces the radioisotope into the labeling precursor compound, in the label introducing unit at a temperature higher than room temperature, to thereby produce the intermediate compound; and
the specific process which is the reaction or the purification of the intermediate compound obtained by the label introducing process.

4. The apparatus for producing a radiolabeled compound according to Claim 3, wherein the specific process is a hydrolysis reaction of the ester group performed in the presence of alkali for the intermediate compound having an ester group.

5. The apparatus for producing a radiolabeled compound according to Claim 3 or 4, further comprising an acid hydrolyzing unit in which an acid hydrolysis reaction of the compound, obtained by the reaction or the purification conducted as the specific process, is carried out under an acidic condition.

6. The apparatus for producing a radiolabeled compound according to any one of Claims 1 to 5, wherein in the specific process,
a compound represented by the formula (2) below is obtained by holding the intermediate compound represented by the formula (1) below in the solid phase extraction unit and passing the alkaline solution through the solid phase extraction unit while cooling the solid phase extraction unit by the cooling unit: 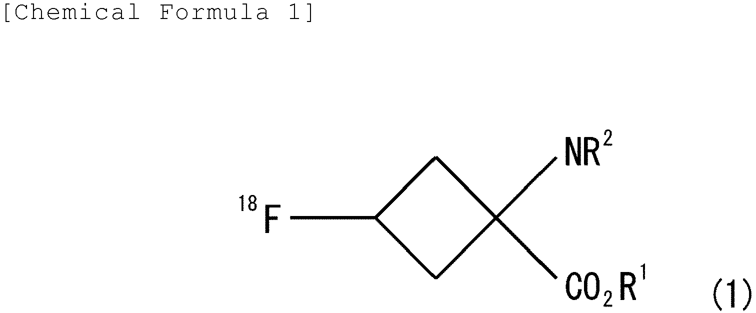 (in the formula, R¹ represents a straight-chain or branched alkyl chain having 1 to 10 carbon atoms or an aromatic substituent, and R² represents a protective group) 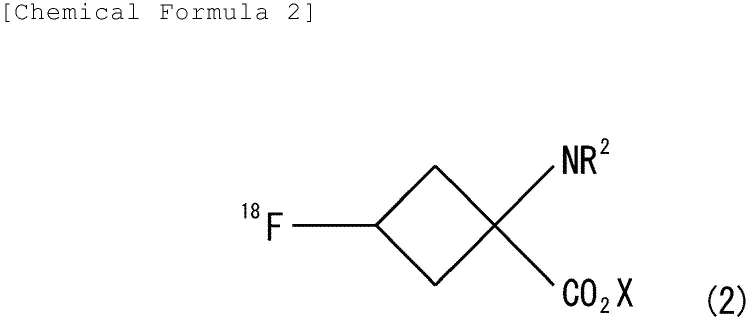 (in the formula, X represents sodium or potassium, and R² represents a protective group).

7. The apparatus for producing a radiolabeled compound according to any one of Claims 1 to 6, wherein the cooling unit contains a cold air blower that cools the solid phase extraction unit with cold air.

8. The apparatus for producing a radiolabeled compound according to Claim 7, wherein the cold air blower includes a vortex tube having an introduction unit that introduces therein compressed air, a cold air output unit that blows out the cold air, and a hot air output unit that blows out hot air,
the vortex tube being disposed so that the hot air output unit blows out the hot air towards the direction opposite to the solid phase extraction unit with reference to the introduction unit.

9. The apparatus for producing a radiolabeled compound according to Claim 7 or 8, further comprising a cover that covers the solid phase extraction unit, and
the cold air blower supplies the cold air inside the cover.

10. The apparatus for producing a radiolabeled compound according to any one of Claims 1 to 9, further comprising a heat sink disposed around the solid phase extraction unit.

11. A method for producing a radiolabeled compound for producing a radiolabeled compound by introducing a radioisotope into a non-radioactive labeling precursor compound, the method comprising:
performing a specific process in a solid phase extraction unit holding an intermediate compound or the radiolabeled compound retained therein, while locally cooling the solid phase extraction unit, the specific process being any one of a reaction of the intermediate compound, a purification of the intermediate compound, or a purification of the radiolabeled compound.

12. The method for producing a radiolabeled compound according to Claim 11, wherein the solid phase extraction unit comprises a solid phase carrier to which a silyl group is bonded, and the specific process is performed in the presence of alkali.

13. The method for producing a radiolabeled compound according to Claim 11 or 12, wherein, in the specific process, a hydrolysis is performed in the presence of alkali for an ester group of the intermediate compound having the ester group, with the surface temperature of the solid phase extraction unit kept at 30°C or below.
